# EUROPEAN PATENT SPECIFICATION

(11) **EP 0 934 754 B1**
(45) Date of publication and mention of the grant of the patent: **19.01.2005**
(21) Application number: 99300428.2
(22) Date of filing: 21.01.1999
(51) Int. Cl.: A61M 5/307, A61M 5/30, C12M 3/00, F17C 7/00

(54) **Needleless medical device for delivering of therapeutic agent**
Nadellose medizinische Vorrichtung zur Verabreichung eines therapeutischen Wirkstoffes
Dispositif médical sans aiguille pour délivrer un agent thérapeutique

(30) Priority: 05.02.1998 GB 9802508
(43) Date of publication of application: 11.08.1999
(73) Proprietor: PowderJect Research Limited, Oxford, OX4 4GA (GB)
(72) Inventor: Surman, David, Guildford, GU2 6YG (GB); Birch, David, Bordon, Hampshire, GU35 9HB (GB)
(74) Representative: Ellis-Jones, Patrick George Armine

(56) References cited:
- EP-A- 0 821 195
- EP-A- 0 888 790
- US-A- 2 628 615
- US-A- 2 680 439
- US-A- 3 115 133
- US-A- 4 717 384
- US-A- 5 630 796

## Description

The present invention relates to needleless medical devices.

Needleless hypodermic injection devices avoid piercing the skin of a patient by employing a fluid, for example, helium at high pressure in the order of 30 - 40 bar to drive a therapeutic agent through the skin of a patient. Such needleless injection devices avoid the discomfort of piercing the epidermis with a hypodermic needle and are of particular comfort to patients with a needle phobia.

In PCT published application WO94/24263 there is described a needleless syringe which includes a metal capsule containing helium gas at high pressure which is used to force particles of a therapeutic agent through the skin of a patient in a substantially painless manner. The capsule is detachable from the remainder of the syringe, and once used, either a new charge of gas can be placed in the capsule or more favourably the capsule can be discarded and a new capsule charged with gas can be attached to the remainder of the syringe.

In European patent publication number 0757202 a needleless medical device is described which includes a capsule for storing a propellant fluid, for example helium under pressure. The capsule is provided with a stopper having a stem formed with a frangible section such that in use mechanical means is employed to rupture the stem about the frangible section to release the helium under high pressure between 60 and 80 bar.

In both these prior art documents, as well as, in EP-A-0 821 195, when the high pressure fluid is released from the capsule it flows directly towards the compartment containing the powdered therapeutic agent. This can be disadvantageous in that the high pressure fluid may contain minute fragments of the capsule generated from that area of the capsule which has been pierced or ruptured.

It is an aim of the present invention to obviate this disadvantage by directing the high pressure fluid initially away from the compartment containing the therapeutic agent.

In US Patent No. 4596556 there is disclosed a device for delivering a therapeutic agent against the skin of a patient which includes a body assembly and a removable capsule assembly. The capsule assembly which is adapted to be partially mounted within the body assembly contains a gas capsule formed with a frangible seal at a proximal end. The proximal end faces in a direction away from a chamber in the capsule assembly containing the therapeutic agent. Mechanical means are mounted on the body assembly adjacent the frangible seal

In use, a projection on the forward end of a firing pin forming part of the mechanical means is driven into the frangible seal such that compressed gases in the capsule explode outwardly in a direction away from the chamber containing the therapeutic agent. This has the effect of driving the capsule and a seal attached to its distal end through the chamber thereby forcing the therapeutic agent from the chamber and through an outlet in the distal end of the capsule assembly towards the skin of a patient.

EP-A-0 888 790 represents prior art according to Article 54(3) EPC.

According to the present invention, there is provided a device according to claim 1.

In some embodiments the capsule has a stem extending outwardly therefrom in a direction towards the fluid release mechanism and away from the second compartment, said stem having a frangible section

In a preferred embodiment a pad of absorbent material is located in the hollow body adjacent the free end of the stem for absorbing any metallic particles that may have been entrained by the escaping fluid from that area of the capsule which is ruptured during the operation of the device.

Embodiments of the invention will now be described, by way of example, reference being made to the Figures of the accompanying diagrammatic drawings in which:-
Figure 1 is a diagrammatic longitudinal cross section through a needleless medical device for delivery a therapeutic agent against the skin of a patient;
Figure 2 is a section on line A-A of Figure 1;
Figure 3 is a diagrammatic longitudinal cross section similar to Figure 1 illustrating a second embodiment of the invention;
Figure 4 is a diagrammatic longitudinal cross section similar to Figure 1 but illustrating yet a further embodiment of the present invention; and
Figure 5 is a partial diagrammatic longitudinal cross-section similar to Figure 1 but illustrating another embodiment of the present invention.

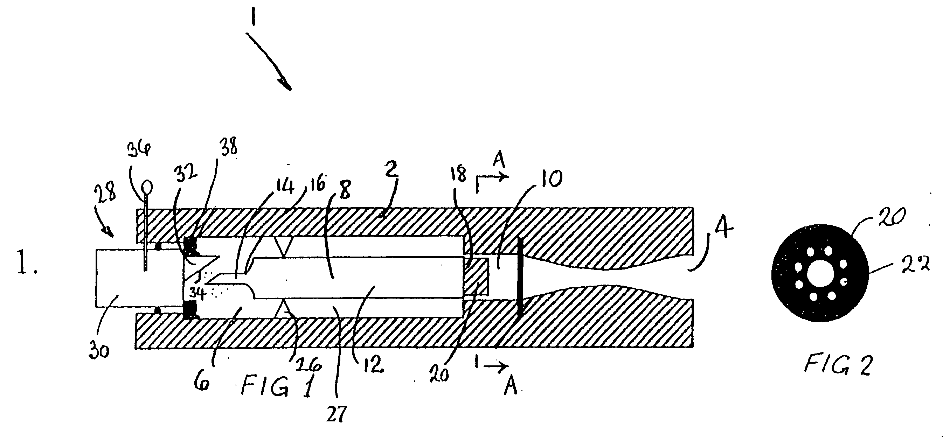

As shown in Figures 1 and 2, a needleless medical device 1 for delivering a therapeutic agent against the skin of a patient comprises essentially an elongate tubular hollow body 2 open at a distal end to define an outlet 4. The hollow body 2 has a first compartment 6 in which is located a gas capsule 8 and a second compartment 10 for receiving the therapeutic agent, for example, a powdered drug.

It will be observed that the second compartment 10 is intermediate the first compartment 6 and the outlet 4.

The gas capsule 8 comprises a hollow body 12 for storing the fluid, for example, helium under high pressure, that is between 60 and 80 bar. A stem 14 extends from the hollow body 12 and includes a frangible section 16. The stem 14 which is hollow is used primarily to fill the hollow body 12 of the capsule 8 with fluid, for example helium under pressure. As shown, the base 18 of the gas capsule 8 is supported by a partition 20 which separates the compartments 6, 10. A plurality of holes 22 extend through the partition 20 to allow the passage of fluid therethrough as will be explained.

The gas capsule 8 is also supported along the length of the hollow body 12 by spaced lugs 26 extending inwardly from the internal surface of the hollow body 2. The outer surface of the gas capsule 8 defines with the inner surface of the hollow body 2 a channel 27.

As shown, the stem 14 extends from the body 12 of the gas capsule 8 in a direction away from the second compartment 10 towards a fluid release mechanism 28 located at the proximal end of the hollow body 2 and which includes a movable button 30 to which is attached a member 32 with an incline surface 34. A safety pin 36 engages the button 30 to prevent accidental actuation of the button 30. A pad 38 of absorbent material is located in the hollow body 2 adjacent the free end of the stem 14.

In use, when it is desired to treat a patient by means of the device 1, the drug is placed in the second compartment 10 and the outlet 4 is placed against the skin of the patient.

Next, the safety pin 36 is withdrawn and finger pressure is applied to the proximal (left hand as shown) end of the button 30 which moves inwardly of the hollow body 2 and causes the inclined surface 34 to engage the free end of the stem 14 thereby rupturing the frangible section 16 with the subsequent release of helium at high pressure. The released helium passes out of the capsule 8 towards the adsorbent pad 38 where any minute particles of metal entrained by the helium as it passes out through the frangible section 16 will be absorbed. The gas is then turned through 180º and passes along the channel 27 between the inside surface of the hollow body 2 and the outside surface of the gas capsule 8 until it reaches the partition 20 when it will pass through the holes 22 to entrain the drug in the compartment 10. Finally the gas with the entrained drug will pass out from the outlet 4 with the drug passing through the skin of the patient whilst the light molecules of helium bounce off the skin into the atmosphere.

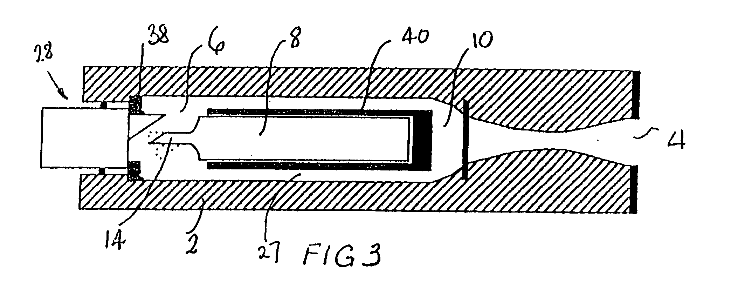

Referring now to Figure 3 where like parts are denoted by like reference numerals the gas capsule 8 is located in the first compartment 6 within a shroud 40 which is formed integral with the hollow body 2. This arrangement has the advantage that the flow of high pressure helium along the channel 27 between the inner surface of the hollow body 2 and the outer surface of the gas capsule 8 will not pick up any contamination from the outer surface of the gas capsule 8. This means that the gas capsule 8 together with the fragile stem 14 does not have to be of high medical cleanliness.

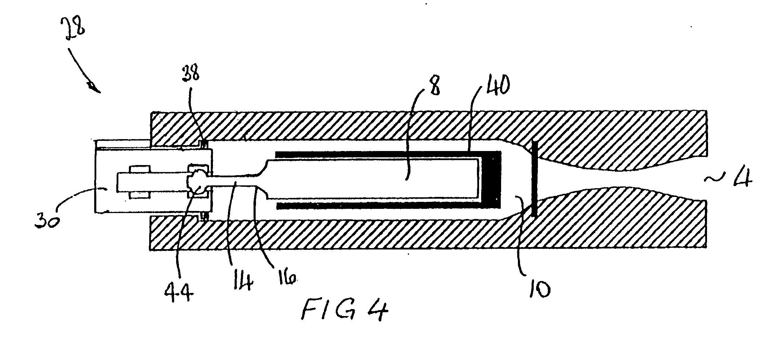

In the embodiment illustrated in Figure 4 where like reference numerals denote like parts the stem 14 of the gas capsule 8 is provided adjacent its free end with a lug 44 which is engaged by the fluid release mechanism 28. The button 30 can be rotated thereby imparting to the lug 44 a twisting motion for shearing off the stem 14 at the frangible section 16.

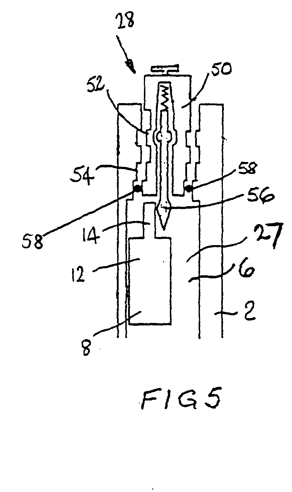

Referring now to Figure 5, where like reference numerals denote like parts; the compartment 6 contains a gas capsule 8 including a hollow body 12 from which extends a stem 14 (upwardly as shown) towards a fluid release mechanism 28. In this embodiment, the stem 14 does not have a frangible section 16 but by contrast the capsule is pierced on operation of the fluid release mechanism 28.

The fluid release mechanism which is located at the proximal (upper as shown) end of the hollow body 2 includes a plunger 50 formed on its outer surface with a screw thread, for example, an archimedean screw thread 52 which co-operates with a complementary screw thread 54 formed on an inner surface of the hollow body 2. Mounted on the plunger 50 for movement there with is a lance 56. A seal 58 ensures a fluid tight seal between the plunger 50 and the internal surface of the hollow body 2. A pad 38 (not shown) may be positioned adjacent the end of the stem 14. In use, turning the plunger 50 will cause the lance 56 to descend thereby piercing or rupturing the capsule 8 with the consequent release of fluid under pressure. The fluid will initially flow towards the proximal end of the hollow body 2 but will then be redirected through 180º towards the second compartment 10 and the patient. The fluid will pass along the channel 27 between the inner surface of the hollow body 2 and the capsule 8 towards the compartment 10. Although not shown, the capsule 8 can be positioned within a shroud 40 as illustrated in Figures 3 and 4.

All the above described embodiments offer the advantage that the high pressure fluid when initially released from the gas capsule 8 will flow in a direction away from the second compartment 10 towards the absorbent pad 38 so that any particles of metal that may be entrained within the fluid are deposited on the absorbent pad thereby providing a clean gas flow for entraining the powdered drug within the second compartment 10.

Further by having the stem 14 facing away from the compartment 10 containing the powdered drug and with the hollow body 12 of the capsule 8 between the stem 14 and the compartment 10, the stem when ruptured cannot be driven through the compartment 10 towards the skin of the patient.

A further advantage of the embodiment illustrated in Figure 5 is that since the stem 14 does not have a frangible section which is ruptured during the release of fluid from the capsule 8 said stem can be made more robust which makes the capsule easier to manufacture and handle.

## Claims

1. A device (1) for delivering a powdered therapeutic agent against the skin of a patient comprising a hollow body (2) open at a distal end to define an outlet (4); a first compartment (6) in the hollow body for containing a capsule (8) in which is stored fluid under pressure and which comprises a hollow stem (14); a second compartment (10) in the hollow body (2) located between the first compartment (6) and the outlet (4) for containing the therapeutic agent; a fluid release mechanism (28) mounted on the hollow body (2) for rupturing the hollow stem (14) or the capsule thereby allowing the escape of fluid from the capsule, **characterised in that** the fluid is allowed to escape initially in a direction away from the second compartment, and **in that** a channel (27) is provided between the outer surface of the capsule (8) and the inner surface of the hollow body (2) to allow for the subsequent flow of the escaped fluid towards the second chamber (10) and the entrainment of the therapeutic agent by said fluid.

2. A device as claimed in claim 1, in which said stem (14) extends outwardly from the capsule (8) in a direction towards the fluid release mechanism (28) and away from the second compartment (10), said stem (14) having a frangible section.

3. A device as claimed in claim 2, in which an absorbent pad (38) is located within the hollow body (2) adjacent the free end of the stem (14) of the capsule.

4. A device as claimed in claim 1, 2 or 3, in which a base (18) of the capsule (8) is supported by a partition (20) located between the first and second compartments, through holes (22) being formed in the partition (20) for fluid communication between the said compartments.

5. A device as claimed in claim 1, 2 or 3, in which the capsule (8) is located within a shroud (40) in the first compartment (6).

6. A device as claimed in any one of claims 2 to 5, in which the fluid release mechanism includes a movable button (30) partially extending from the proximal end of the hollow body (2) and to which is attached a member (32) formed with an inclined surface (34), the arrangement being such that pressure on the proximal surface of the button causes the inclined surface (34) to engage the free end of the stem (14) thereby causing the frangible section to rupture.

7. A device as claimed in claim 6, in which a safety pin (36) is provided to prevent accidental actuation of the button (30).

8. A device as claimed in any one of claims 2 to 5 in which the stem (14) is formed at or adjacent its free end with a lug (44) and the fluid release mechanism (28) includes a rotatable button (30) which engages said lug, the arrangement being such that rotation of the button imparts a twisting motion to the lug thereby shearing the stem (14) at the frangible section.

9. A device as claimed in claim 1 in which the fluid release mechanism (28) includes a plunger (50) the rotation of which causes a lance (56) to pierce/rupture the capsule.

## Patentansprüche

1. Vorrichtung (1) für das Zuführen eines Therapeutikums in Pulverform gegen die Haut eines Patienten, welche umfasst: einen Hohlkörper (2), welcher an einem distalen Ende offen ist, so dass er einen Auslass (4) bildet; eine erste Kammer (6) in dem Hohlkörper für das Aufnehmen einer Kapsel (8), in welcher Fluid unter Druck gespeichert ist und welche einen hohlen Schaft (14) umfasst; eine in dem Hohlkörper (2) zwischen der ersten Kammer (6) und dem Auslass (4) angeordnete zweite Kammer (10) für das Aufnehmen des Therapeutikums; einen an dem Hohlkörper (2) angebrachten Fluidabgabemechanismus (28) für das Zerbrechen des hohlen Schafts (14) oder der Kapsel, wodurch ein Austreten von Fluid aus der Kapsel zugelassen wird, **dadurch gekennzeichnet, dass** das Fluid anfänglich in eine Richtung weg von der zweiten Kammer austreten kann und dass ein Kanal (27) zwischen der Außenfläche der Kapsel (8) und der Innenfläche des Hohlkörpers (2) vorgesehen ist, um den anschließenden Fluss des ausgetretenen Fluids hin zur zweiten Kammer (10) und das Mitführen des Therapeutikums durch das Fluid zu ermöglichen.

2. Vorrichtung nach Anspruch 1, bei welcher der Schaft (14) von der Kapsel (8) nach außen in eine Richtung hin zum Fluidabgabemechanismus (28) und weg von der zweiten Kammer (10) erstreckt, wobei der Schaft (14) einen zerbrechlichen Abschnitt aufweist.

3. Vorrichtung nach Anspruch 2, bei welcher ein absorbierender Polster (38) in dem Hohlkörper (2) neben dem freien Ende des Schafts (14) der Kapsel angeordnet ist.

4. Vorrichtung nach Anspruch 1, 2 oder 3, bei welcher ein Boden (18) der Kapsel (8) durch eine zwischen der ersten und der zweiten Kammer angeordnete Abtrennung (20) gelagert wird, wobei in der Abtrennung (20) durchgehende Öffnungen (22) für die Fluidverbindung zwischen den Kammern ausgebildet sind.

5. Vorrichtung nach Anspruch 1, 2 oder 3, bei welcher die Kapsel (8) in einer Hülle (40) in der ersten Kammer (6) angeordnet ist.

6. Vorrichtung nach einem der Ansprüche 2 bis 5, bei welcher der Fluidabgabemechanismus einen sich teilweise von dem proximalen Ende des Hohlkörpers (2) erstreckenden beweglichen Knopf (30) umfasst, an welchem ein mit einer geneigten Fläche (34) ausgebildetes Element (32) befestigt ist, wobei die Anordnung solcher Art ist, dass Druck an der proximalen Fläche des Knopfes ein Greifen der geneigten Fläche (34) mit dem freien Ende des Schafts (14) bewirkt, wodurch ein Zerbrechen des zerbrechlichen Abschnitts bewirkt wird.

7. Vorrichtung nach Anspruch 6, bei welcher ein Sicherheitsstift (36) vorgesehen ist, um eine versehentliche Betätigung des Knopfes (30) zu verhindern.

8. Vorrichtung nach einem der Ansprüche 2 bis 5, bei welcher der Schaft (14) an oder neben seinem freien Ende mit einer Nase (44) ausgebildet ist und der Fluidabgabemechanismus (28) einen drehbaren Knopf (30) umfasst, welche mit der Nase greift, wobei die Anordnung solcher Art ist, dass die Drehung des Knopfes eine Verdrehbewegung auf die Nase ausübt, wodurch der Schaft (14) an dem zerbrechlichen Abschnitt abgetrennt wird.

9. Vorrichtung nach Anspruch 1, bei welcher der Fluidabgabemechanismus (28) einen Stößel (50) umfasst, dessen Drehung eine Lanze (56) veranlasst, die Kapsel zu durchstechen/zerbrechen.

## Revendications

1. Dispositif (1) pour administrer un agent thérapeutique en poudre contre la peau d'un patient, comprenant un corps creux (2) ouvert à une extrémité distale pour définir une sortie (4) ; un premier compartiment (6) dans le corps creux pour contenir une capsule (8) dans laquelle est stocké un fluide sous pression, et qui comporte une tige creuse (14) ; un deuxième compartiment (10) dans le corps creux (2) situé entre le premier compartiment (6) et la sortie (4) pour contenir l'agent thérapeutique ; un mécanisme (28) de libération de fluide monté sur le corps creux (2) pour briser la tige creuse (14) ou la capsule en laissant de ce fait s'échapper le fluide de la capsule, **caractérisé en ce que** le fluide est autorisé à s'échapper initialement dans une direction opposée au deuxième compartiment, et **en ce qu'**un canal (27) est présent entre la surface extérieure de la capsule (8) et la surface intérieure du corps creux (2) pour permettre l'écoulement ultérieur du fluide échappé vers la deuxième chambre (10) et l'entraînement de l'agent thérapeutique par ledit fluide.

2. Dispositif selon la revendication 1, dans lequel ladite tige (14) s'étend vers l'extérieur de la capsule (8) en direction du mécanisme (28) de libération de fluide et à l'opposé du deuxième compartiment (10), ladite tige (14) ayant une partie cassante.

3. Dispositif selon la revendication 2, dans lequel un tampon absorbeur (38) est situé dans le corps creux (2) au voisinage immédiat de l'extrémité libre de la tige (14) de la capsule.

4. Dispositif selon la revendication 1, 2 ou 3, dans lequel une embase (18) de la capsule (8) est supportée par une cloison (20) située entre le premier et le deuxième compartiments, des trous traversants (22) étant ménagés dans la cloison (20) pour une communication de fluide entre lesdits compartiments.

5. Dispositif selon la revendication 1, 2 ou 3, dans lequel la capsule (8) est logée dans une enveloppe de protection (40) dans le premier compartiment (6).

6. Dispositif selon l'une quelconque des revendications 2 à 5, dans lequel le mécanisme de libération de fluide comporte un bouton mobile (30) s'étendant partiellement depuis l'extrémité proximale du corps creux (2) et auquel est fixé un élément (32) pourvu d'une surface inclinée (34), l'agencement étant tel qu'une pression sur la surface proximale du bouton fait venir la surface inclinée (34) contre l'extrémité libre de la tige (14) en provoquant de la sorte la rupture de la partie cassante.

7. Dispositif selon la revendication 6, dans lequel une goupille de sécurité (36) est prévue pour empêcher l'actionnement accidentel du bouton (30).

8. Dispositif selon l'une quelconque des revendications 2 à 5, dans lequel la tige (14) est pourvue, à ou au voisinage immédiat de son extrémité libre, d'une oreille (44), et le mécanisme (28) de libération de fluide comporte un bouton tournant (30) qui coopère avec ladite oreille, l'agencement étant tel que la rotation du bouton transmet un mouvement de torsion à l'oreille en cisaillant de ce fait la tige (14) au niveau de la partie cassante.

9. Dispositif selon la revendication 1, dans lequel le mécanisme (28) de libération de fluide comporte un plongeur (50) dont la rotation amène une lance (56) à percer/briser la capsule.
